# EUROPÄISCHE PATENTSCHRIFT

(11) **EP 0 834 312 B1**
(45) Veröffentlichungstag und Bekanntmachung des Hinweises auf die Patenterteilung: **08.01.2003**
(21) Anmeldenummer: 97117282.0
(22) Anmeldetag: 06.10.1997
(51) Int. Cl.: A61K 31/195, A61K 9/70, A61K 47/24, A61K 47/10

(54) **Topisches Arzneimittel auf Basis von Diclofenac**
Topical medicament containing diclofenac
Préparation topique à base de diclofenac

(30) Priorität: 07.10.1996 DE 19641259
(43) Veröffentlichungstag der Anmeldung: 08.04.1998
(73) Patentinhaber: Dr. Kade Pharmazeutische Fabrik GmbH, 12277 Berlin (DE)
(72) Erfinder: Müller, Gerhard, 78464 Konstanz (DE)
(74) Vertreter: Kinzebach, Werner, Dr.

(56) Entgegenhaltungen:
- EP-A- 0 372 527
- EP-A- 0 704 206
- WO-A-95/05163
- DE-A- 4 313 402
- US-A- 5 374 661
- PATENT ABSTRACTS OF JAPAN vol. 096, no. 004, 30.April 1996 & JP 07 316075 A (POLA CHEM IND INC), 5.Dezember 1995,
- PATENT ABSTRACTS OF JAPAN vol. 016, no. 560 (C-1008), 2.Dezember 1992 & JP 04 217925 A (NIHON SAAFUAKUTANTO KOUGIYOU KK), 7.August 1992,

## Beschreibung

Die vorliegende Erfindung betrifft ein Diclofenac enthaltendes Arzneimittel zur topischen Behandlung von Entzündungen und Schmerzen.

Diclofenac (2-(2,6-Dichloranilino)phenylessigsäure) hat aufgrund seiner ausgezeichneten entzündungshemmenden, analgetischen und antipyretischen Eigenschaften als nichtsteroidales Antirheumatikum in der Pharmazie große Bedeutung erlangt. Die orale Anwendung dieses Wirkstoffes ist allerdings wegen häufig auftretender, zum Teil gravierender Nebenwirkungen, wie Magen-Darm-Blutungen oder Nierenfunktionsschädigungen, nicht unbedenklich.

Oft ist es daher wünschenswert, die orale durch eine topische Anwendung zu ersetzen. Dies ist insbesondere dann sinnvoll, wenn die zu behandelnden Erkrankungen oder Verletzungen örtlich lokalisierbar und durch eine topische Anwendung leicht zugänglich sind. Hierzu zählen beispielsweise Erkrankungen und Verletzungen des Bewegungsapparates oder peripherer Weichteile.

Ein Problem bei der Konzipierung topisch anzuwendender Arzneimittel ist, daß die Haut eine sehr effektive Penetrationsbarriere darstellt. Die Epidermis weist eine äußere Schicht dichtgepackter toter Zellen, das sogenannte Stratum corneum, auf, welches das Eindringen gasförmiger, fester oder flüssiger Substanzen als solche oder in wäßriger oder öliger Lösung behindert. Hat ein pharmazeutischer Wirkstoff erst einmal das Stratum corneum passiert, kann er leicht in tiefere Schichten vordringen.

Aus DE 33 36 047 ist bereits ein Diclofenac enthaltendes topisches Präparat bekannt, das aber eine Öl/Wasser-Emulsion ist und Niederalkanole enthält.

EP-A-0488089 beschreibt ebenfalls topische Diclofenac-Emulsionen mit erheblichen Anteilen eines flüssigen Öls.

EP-A-0372527 beschreibt topische Diclofenac-Präparate, die Hydroxyethylpyrrolidin enthalten.

EP-A-0704206 beschreibt topische Präparate, die u. a. auch Diclofenac enthalten können. Diese Präparate sind jedoch so zusammengesetzt, daß sie in wenigen Sekunden nach Applikation den Wirkstoff in Form einer bestimmten Feinverteilung, nämlich als Liposomen, und in erhöhter Konzentration durch rasches Verdampfen eines Niederalkanols auf der Hautoberfläche bereitstellen sollen.

All diese Präparate wirken nicht rasch genug und verursachen häufig Hautreizungen.

Ferner wird in DE 43 13 402 A1 eine transdermale Wirkstoffzubereitung mit einem Gehalt an Phospholipid und Alkohol beschrieben, deren Permeationsvermögen durch den Zusatz von α-Tocopherol oder α-Tocopherol-Derivaten verbessert werden soll.

JP 07 316075A betrifft analgetische Formulierungen zur externen Anwendung, die der Haut feuchtigkeitsrückhaltende Funktion verleihen sollen.

Die in WO 95/05163 beschriebenen Öl-in-Wasser-Emulsionen sind speziell zur Bioadhäsion an Schleimhäuten konzipiert.

Wirkstoff, Öl, Wasser und ein wasserlösliches Polyol enthaltende Zusammensetzungen werden in JP 04 217925 vorgeschlagen.

Die in US-A-5,374,661 angegebene topische Wirkstoffzubereitung enthält Diclofenac-Natrium, das in einem Gemisch aus Wasser, einem niedermolekularen Alkohol und Glykol solubilisiert ist. So enthalten die Zubereitungen vorzugsweise 10 bis 20 Gew.-% Ethanol, Isopropanol und ähnliche, bei chronischer Anwendung die Haut austrocknende Alkohole. Zur Erhöhung der transdermalen Permeation können Ether von Alkoholen und Ester von Fettalkoholen zugesetzt werden.

Der Erfindung liegt demgegenüber die Aufgabe zugrunde, ein pharmazeutisches Mittel zur topischen Anwendung von Diclofenac oder eines pharmazeutisch verträglichen Salzes davon bereitzustellen, das rasch wirkt und daher den Wirkstoff sehr gut durch die Haut penetrieren lässt, gut verträglich ist, über eine ausreichende Lagerstabilität verfügt und sich durch eine leichte und verläßliche Handhabung auszeichnet.

Es wurde nun überraschenderweise gefunden, daß diese Aufgabe durch ein Diclofenac enthaltendes, topisches Mittel mit mindestens einem Diethylenglycolmonoalkylether umfassenden Lösungsmittel und mindestens einem Lösungsvermittler gelöst wird, das als Lösungsvermittler mindestens ein Phospholipid enthält.

Gegenstand der vorliegenden Erfindung ist daher ein Arzneimittel auf Basis von Diclofenac oder eines seiner pharmazeutisch verträglichen Salze zur topischen Behandlung von Entzündungen und Schmerzen mit mindestens einem Lösungsmittel und mindestens einem Lösungsvermittler, das dadurch gekennzeichnet ist, daß es als Lösungsvermittler mindestens ein Phospholipid enthält, und dass das Lösungsmittel ein Gemisch aus Wasser, Diethylenglycolmonoethylether und gegebenenfalls C₂-C₆-Polyalkoholen sowie Polyethern davon, deren Estern und Ethern, sowie Glyceriden und/oder ethoxylierten Derivaten davon ist.

Das erfindungsgemäße Mittel enthält Diclofenac bzw. ein pharmazeutisch verträgliches Salz davon. Bevorzugt sind Salze der Säure mit Alkalimetallbasen, insbesondere das Natriumsalz, oder Salze mit einer quaternären Ammoniumverbindung sowie Säureadditionssalze von Diclofenac mit in der Pharmazie üblicherweise verwendeten basischen Stickstoffverbindungen, insbesondere primären, sekundären oder tertiären Aminen, primären, sekundären oder tertiären Säureamiden oder Imiden.

Das erfindungsgemäße Mittel enthält Diclofenac oder dessen Salz in gelöster Form in einem geeigneten pharmazeutisch verträglichen Lösungsmittel, das einen oder mehrere geeignete pharmazeutisch verträgliche Lösungsvermittler enthält.

Als Lösungsmittel verwendet man ein wasserhaltiges Lösungsmittelgemisch.

Unter Lösungsvermittler versteht man im Rahmen der vorliegenden Erfindung eine Substanz, welche die Löslichkeit des Wirkstoffes in dem Lösungsmittel erhöht. Erfindungsgemäß eignen sich zu diesem Zweck vorzugsweise amphiphile Lipide. Derartige Substanzen besitzen einen hydrophoben Teil, der durch eine lipidartige Komponente gebildet wird, und einen hydrophilen Teil, der anionischer, kationischer, zwitterionischer oder auch nichtionischer polarer Natur sein kann.

Von den Phospholipiden sind natürliche Phospholipide, beispielsweise Lecithine, wie Saflor-, Raps- und insbesondere Ei- oder Sojalecithine, enzymatisch oder chemisch modifizierte Phospholipide, beispielsweise hydriertes Lecithin, und synthetische Phospholipide, beispielweise Dipalmitoylphosphatidylcholin, erfindungsgemäß bevorzugt.

Der Begriff Lecithin umfasst in der Regel ein Gemisch aus verschiedenen Phospholipiden und weiteren Lipidkomponenten. Neben den Hauptbestandteilen Phosphatidylcholin und Phosphatidylethanolamin findet man üblicherweise Lysophosphatidylcholin, Lysophosphatidylethanolamin, Phosphatidylserin und Phosphatidylinosit. Zu den weiteren Lipidkomponenten zählen verschiedene Phytoglycolipide, Phosphatidsäuren, Sphingomyelin und zum Teil erhebliche Anteile an weiteren Begleitlipiden. An Fettsäuren findet man hauptsächlich Stearin-, Palmitin-, Öl-, Linol-, Linolen- und Arachidonsäure.

Erfindungsgemäß besonders bevorzugte Phospholipide sind fettfreie Lecithine mit Anteilen von 10-100 % Phosphatidylcholin.

Neben wenigstens einem der oben beschriebenen Phospholipide kann das erfindungsgemäße Mittel weitere Lösungsvermittler enthalten.

Es eignen sich kationische amphiphile Lipide, von denen in erster Linie quaternäre Ammoniumsalze zu nennen sind. Derartige Ammoniumverbindungen weisen bevorzugt zwei langkettige C₁₂-C₂₀-Alkylreste und zwei kurzkettige C₁-C₄-Alkylreste auf.

Eine weitere Gruppe erfindungsgemäß geeigneter Lösungsvermittler bilden nichtionische Verbindungen, die - wie oben beschrieben - einen lipidartigen und einen polaren Teil aufweisen. Der lipidartige Teil wird in der Regel von einem langkettigen, linearen oder verzweigten, gesättigten oder ungesättigten, aliphatischen Rest mit 12 bis 30 Kohlenstoffatomen oder einem entsprechenden Acylrest gebildet. Es ist allerdings auch möglich, daß sie anstelle der Kette eine Ringverbindung aufweisen, beispielsweise Sterole und Sterolderivate. Der polare Teil weist in der Regel eine oder mehrere, O- oder N-enthaltende funktionelle Gruppen auf, beispielsweise freie Hydroxylgruppen, Ether-, Ester-, Amid- oder Imidgruppen.

Bevorzugte Verbindungen der obengenannten Art sind Ester und/oder Ether von Polyolen, die gegebenfalls ethoxyliert sein können. Als Polyol wird insbesondere Sorbitan oder Polyethylenglycol verwendet. Erfindungsgemäß geeignet sind in diesem Zusammenhang sowohl Verbindungen, die vollständig verestert und/oder verethert sind, als auch Verbindungen, die noch freie Hydroxylgruppen aufweisen, sogenannte Partialester oder -ether.

Ferner eignen sich erfindungsgemäß gegebenenfalls ethoxylierte Sterole oder Phytosterole, beispielsweise Cholesterin, Glycolipide natürlichen oder synthetischen Ursprungs oder gegebenenfalls ethoxylierte Fettamine.

Insbesondere bevorzugt von den nichtionischen amphiphilen Lipiden sind die Polyethylenglycolsorbitanfettsäureester, Sorbitanfettsäureester, Polyethylenglycolfettsäureester, Polyethylenglycolfettalkoholether und Partialfettsäureester von Polyolen. Diese bilden mit den erfindungsgemäßen Phospholipiden vorteilhafte Lösungsvermittlergemische.

Ethoxylierte Verbindungen weisen erfindungsgemäß 1 bis 40 Ethylenoxideinheiten auf.

Erfindungsgemäße Lösungsmittel basieren auf Wasser und Diethylenglycolmonoethylether. Sie enthalten eine oder mehrere Verbindungen, welche die Löslichkeit des Wirkstoffes und seine topische Anwendung auf der Haut in günstiger Weise beeinflussen. Hierzu zählen C₂-C₆-Polyalkohole sowie Polyether davon, wie Ethylenglycol, Diethylenglycol, Propylenglycol, Glycerin, Sorbitol, Polyethylenglykol oder Polypropylenglykol, deren Ester und Ether, insbesondere Diethylenglycolmonoalkyl-ether, die auch als Ethyl(en)diglycolmonoalkylether bezeichnet werden können, wie Diethylenglycolmonomethylether, Diethylenglycolmonobutylether und besonders bevorzugt Diethylenglycolmonoethylether, sowie Glyceride und ethoxylierte Derivate davon, vorzugsweise gesättigte, polyglycolisierte Glyceride.

Erfindungsgemäße Lösungsmittel stellen ein Gemisch aus Wasser, Diethylenglycolmonoethylether und gegebenefalls einem oder mehreren der vorstehend genannten Substanzen dar. Besonders bevorzugt sind Gemische aus Propylenglycol und Diethylenglycolmonoethylether mit Wasser. Ihr Anteil am Gesamtgewicht des Mittels kann bis zu 70 Gew.-% betragen und liegt vorzugsweise bei 40 bis 70 Gew.-%.

Der Ausdruck "Alkyl" umfasst geradkettige oder verzweigte Alkylgruppen, wie Methyl, Ethyl, n- und i-Propyl, n- ,i- oder t-Butyl, n-Pentyl, Neopentyl, n-Hexyl etc. Soweit nicht anders angegeben, steht "Alkyl" in den vorstehend genannten Resten, vorzugsweise für C₁-C₆-Alkyl und besonders bevorzugt für C₁-C₄-Alkyl.

Der Begriff "Polyol" steht für "Polyalkohole" und bezeichnet erfindungsgemäß lineare oder verzweigte Alkylreste mit wenigstens zwei Hydroxylgruppen und vorzugsweise 2-8 Kohlenstoffatomen sowie deren Polyether. Beispiele für lineare Reste sind Ethylenglycol, Diethylenglycol, Polyethylenglycol, Propylenglycol, Polypropylenglycol, Glycerin, Erythrol, Pentaerythrol, Sorbitol, Sorbitan sowie deren Isomere. Zu den verzweigten Resten zählt beispielsweise Trimethylolpropan.

Unter dem Präfix "Fett-" versteht man erfindungsgemäß lineare oder verzweigte, gesättigte oder ungesättigte, langkettige Alkylreste, vorzugsweise mit 12 bis 30 und insbesondere mit 12 bis 24 Kohlenstoffatomen. Tragen diese Reste eine Carboxylgruppe, spricht man von Fettsäuren, beispielsweise Laurin-, Myristin-, Palmitin-, Stearin-, Arachinin-, Behenin-, Lignocerin-, Palmitolein-, Öl-, Linol-, Linolen- oder Arachidonsäure. Aus solchen Fettsäuren lassen sich durch Reduktion der Carboxylfunktion die entsprechenden Fettalkohole ableiten.

Das erfindungsgemäße Mittel enthält 0,5 bis 20 Gew.-%, vorzugsweise 1 bis 10 Gew.-% und insbesondere etwa 5 Gew.-% Diclofenac und/oder dessen Salz.

Der auf das Gesamtgewicht des Mittels bezogenene Anteil an Lösungsvermittler beträgt in der Regel 0,5 bis 25 Gew.-%, vorzugsweise 1 bis 10 Gew.-% und insbesondere etwa 3 Gew.-%. Der auf das Gesamtgewicht des Mittels bezogenene Anteil an Phospholipid beträgt in der Regel 0,5 bis 20 Gew.-%, vorzugsweise 1 bis 5 Gew.-% und insbesondere etwa 2 Gew.-%.

Das erfindungsgemäße Mittel kann weitere für derartige Zusammensetzungen übliche pharmazeutische Hilfs- und/oder Trägerstoffe enthalten, insbesondere Mittel zur pH-Einstellung, Stabilisatoren, Konservierungsmittel, wie Benzalkoniumchlorid oder Phenoxyethanol, Antioxidantien, wie α-Tocopherol, Rückfettungsmittel und/oder Parfüms.

Gegebenenfalls kann das erfindungsgemäße Mittel neben dem eigentlichen Wirkstoff Diclofenac oder dessen Salz weitere, mit Diclofenac im Rahmen der vorliegenden Anwendung verträgliche pharmazeutische Wirkstoffe enthalten. Beispiele sind Hautpflegemittel, Desinfektionsmittel, Antibiotika, Lokalanästhetika, durchblutungsfördernde Mittel oder etherische Öle.

Die Herstellung des erfindungsgemäßen Mittels erfolgt auf herkömmliche Art und Weise, indem man die Komponenten miteinander vermischt. Beispielsweise kann man den Wirkstoff in Lösungsmittel auflösen und die so erhaltenen Phase mit Wasser und gegebenfalls in Lösungsmittel gelöstem Lösungsvermittler vermischen. So kann der Wirkstoff in Diethylenglycolmonoethylether gelöst werden, während Lecithin zunächst mit Propylenglycol vermischt wird. Diese beiden Phasen können dann unter Zugabe von Wasser miteinander vereinigt werden.

Das erfindungsgemäße Mittel wird vorzugsweise als Lösung zum Einreiben, als Hautspray, Gel oder getränktes Pflaster zubereitet und angeboten. Besonders vorteilhaft ist ein Hautspray, insbesondere ein Pumpspray.

Im Rahmen der vorliegenden Erfindung versteht man unter Spray eine galenische Zubereitungsform, bei der das Arzneimittel im Augenblick der Anwendung einen feinen Sprühnebel, insbesondere ein Aerosol bildet. Aerosole stellen disperse Systeme dar, von denen erfindungsgemäß die Nebelaerosole (flüssig/gasförmig) bevorzugt werden. Die zur Konfektionierung von Sprays notwendigen Bestandteile, wie Behältnisse, Ventilsysteme, Handpümpchen oder geeignete Treibmittel sind dem Galeniker hinlänglich bekannt. Vorzugsweise wird das Mittel als Pumpspray zubereitet.

Eine weitere vorteilhafte Darbietungsform ist die als Hydrogel. Zu diesem Zweck gibt man einen Gelbildner zu der hydrophilen Phase des Mittels, so daß eine streichfähige Zubereitung entsteht.

Als Gelbildner eignen sich z.B.:
a) Anorganische Gelbildner, wie
   - hochdisperses Siliciumdioxid sowie dessen Mischungen mit Aluminiumoxid;
   - Magnesium-Aluminiumsilikate, z.B. Bentonite, Veegum®;
b) organische Gelbildner, wie
   - native Substanzen, wie Gelatine, Polysaccharide (z.B. Stärke, Pektin, Tragant, Alginate, Xanthangummi);
   - halbsynthetische Gelbildner, wie Celluloseether (z.B. Methylcellulose, Ethylcellulose, Hydroxyethyl-cellulose, Hydroxypropylcellulose, Hydroxypropyl-methylcellulose, Natriumcarboxymethylcellulose), Stärkederivate, Pektinderivate u.a;
   - vollsynthetische Gelbildner, wie Polyvinylalkohol, Polyvinylpyrrolidone u.a.

Eine vorteilhafte Ausführungsform der vorliegenden Erfindung betrifft ein Mittel, das Diclofenac-Natrium, Phospholipid, Diethylenglycolmonoethylether, Propylenglykol und gereinigtes Wasser umfasst. Gemäß einer weiteren vorteilhaften Ausführungsform enthält das Mittel auch einen Puffer, beispielsweise einen Natriumhydrogenphosphat-Puffer mit einem pH von etwa 6 bis 7, ein Antioxidans und ein Konservierungsmittel.

Eine besonders vorteilhafte Ausführungsform der vorliegenden Erfindung betrifft ein Mittel, das 5 bis 15 Gew.-% und insbesondere etwa 8 bis 12 Gew.-% Propylenglycol, 10 bis 30 Gew.-% und insbesondere etwa 20 bis 25 Gew.-% Diethylenglycolmonoethylether, sowie 20 bis 60 Gew.-% und insbesondere 35 bis 45 Gew.-% gereinigtes Wasser umfasst. Derartige Lösungsmittelgemische eignen sich hervorragend in Kombination mit 0.5 bis 5 Gew.-% und insbesondere etwa 1 bis 3 Gew.-% Phospholipid zur Formulierung von Mitteln, die vorzugsweise 4 bis 7 und insbesondere etwa 5 Gew.-% Diclofenac-Natrium umfassen. Ferner sind 10 bis 30 Gew.-% und insbesondere 15 bis 25 Gew.-% Puffer von Vorteil.

Das folgende Beispiel beschreibt die Erfindung, ohne sie zu beschränken.

### Beispiel 1:

| **Spray mit 5 % Diclofenac-Natrium** | |
|---|---|
| Diclofenac-Natrium | 50,0 mg |
| DL-α-Tocopherol | 0,3 mg |
| Phenoxyethanol | 1,0 mg |
| Lecithin | 30,0 mg |
| Propylenglycol | 80,0 mg |
| Natriumdihydrogenphosphat-Puffer pH 6,3 | 100,0 mg |
| Diethylenglycolmonoethylether (Transcutol®) | 300,0 mg |
| gereinigtes Wasser | 438,7 mg |

Falls gewünscht, kann diese Zusammensetzung noch parfümiert werden. Das so erhaltene Gemisch wird in üblicher, dem Galeniker bekannter Art und Weise zu einem Spray konfektioniert.

Die topische Anwendung dieses Sprays zeigt eine überraschend hohe Wirksamkeit. Im Hautpenetrationstest ist es einem im Handel befindlichen Vergleichspräparat in Form eines Geles (Voltaren Emulgel) deutlich überlegen.

## Patentansprüche

1. Arzneimittel auf Basis von Diclofenac oder eines seiner pharmazeutisch verträglichen Salze zur topischen Behandlung von Entzündungen und Schmerzen mit einem Lösungsmittel und mindestens einem Lösungsvermittler, **dadurch gekennzeichnet, daß** es als Lösungsvermittler mindestens ein Phospholipid enthält, und dass das Lösungsmittel ein Gemisch aus Wasser, Diethylenglycolmonoethylether und gegebenenfalls C₂-C₆-Polyalkoholen sowie Polyethern davon, deren Estern und Ethern, sowie Glyceriden und/oder ethoxylierten Derivaten davon ist.

2. Mittel nach Anspruch 1, **dadurch gekennzeichnet, daß** das Phospholipid ein natürliches Phospholipid, insbesondere Ei- oder Sojalecithin, enzymatisch oder chemisch modifiziertes Phospholipid, insbesondere hydriertes Lecithin, oder ein synthetisches Phospholipid, insbesondere Dipalmitoylphosphatidylcholin, ist.

3. Mittel nach Anspruch 1 oder 2, **dadurch gekennzeichnet, daß** das Phospholipid fettfrei ist.

4. Mittel nach einem der vorhergehenden Ansprüche, **dadurch gekennzeichnet, dass** es Phospholipid in einer Menge von 0,5 bis 20 Gew.-%, bezogen auf das Gesamtgewicht des Mittels, enthält.

5. Mittel nach einem der vorhergehenden Ansprüche, **dadurch gekennzeichnet, daß** es als weiteren Lösungsvermittler mindestens einen der folgenden Stoffe, gegebenenfalls ethoxylierte Ester und Ether von Polyolen, insbesondere Polyethylenglycolsorbitanfettsäureester, Sorbitanfettsäureester, Polyethylenglycolfettsäureester, Polyethylenglycolfettalkoholether und Partialfettsäureester von Polyolen, enthält.

6. Mittel nach einem der vorhergehenden Ansprüche, **dadurch gekennzeichnet, dass** es Lösungsvermittler in einer Menge von 0,5 bis 25 Gew.-%, bezogen auf das Gesamtgewicht des Mittels, enthält.

7. Mittel nach einem der vorhergehenden Ansprüche, **dadurch gekennzeichnet, dass** das Lösungsmittelgemisch Ethylenglycol, Diethylenglycol, Propylenglycol, Glycerin, Sorbitol, Polyethylenglycol und/oder Polypropylenglycol enthält.

8. Mittel nach Anspruch 7, **dadurch gekennzeichnet, daß** das Lösungsmittel ein Gemisch aus 20 bis 60 Gew.-% Wasser, 5 bis 15 Gew.-% Propylenglycol und 10 bis 30 Gew.-% Diethylenglycolmonoethylether, jeweils auf das Gesamtgewicht der Mittel bezogen, ist.

9. Mittel nach einem der vorhergehenden Ansprüche, **dadurch gekennzeichnet, daß** es Diclofenac oder dessen Salz, vorzugsweise Diclofenac-Natrium, in einer Menge von 0,5-20 Gew.-%, bezogen auf das Gesamtgewicht des Mittels, enthält.

10. Mittel nach einem der vorhergehenden Ansprüche, **dadurch gekennzeichnet, dass** es weitere übliche pharmazeutische Hilfs- und/oder Trägerstoffe sowie gegebenenfalls weitere Wirkstoffe enthält.

11. Mittel nach einem der vorhergehenden Ansprüche in Form einer Lösung zum Einreiben, eines Hautsprays, Geles oder eines getränkten Pflasters.

12. Verfahren zur Herstellung eines Mittels nach einem der vorhergehenden Ansprüche, wobei man Diclofenac oder eines seiner pharmazeutisch verträglichen Salze in Lösungsmittel auflöst, und die so erhaltene Phase mit Wasser und gegebenenfalls in Lösungsmittel gelöstem Lösungsvermittler vermischt.

## Claims

1. Pharmaceutical composition based on diclofenac or one of the pharmaceutically acceptable salts thereof for the topical treatment of inflammation and pain, comprising a solvent and at least one solubiliser, **characterised in that** it contains as solubiliser at least one phospholipid, and **in that** the solvent is a mixture of water, diethyleneglycol monoethyl ether and optionally C₂₋₆-polyalcohols and polyethers thereof, the esters and ethers thereof, as well as glycerides and/or ethoxylated derivatives thereof.

2. Composition according to claim 1, **characterised in that** the phospholipid is a natural phospholipid, particularly egg or soya lecithin, an enzymatically or chemically modified phospholipid, particularly hydrogenated lecithin, or a synthetic phospholipid, particularly dipalmitoyl phosphatidylcholine.

3. Composition according to claim 1 or 2, **characterised in that** the phospholipid is fat-free.

4. Composition according to one of the preceding claims, **characterised in that** it contains phospholipid in an amount of from 0.5 to 20 wt.%, based on the total weight of the composition.

5. Composition according to one of the preceding claims, **characterised in that** it contains as additional solubiliser at least one of the following substances: optionally ethoxylated esters and ethers of polyols, particularly polyethyleneglycol sorbitan fatty acid esters, sorbitan fatty acid esters, polyethyleneglycol fatty acid esters, polyethyleneglycol fatty alcohol ethers and partial fatty acid esters of polyols.

6. Composition according to one of the preceding claims, **characterised in that** it contains solubiliser in an amount of from 0.5 to 25 wt.%, based on the total weight of the composition.

7. Composition according to one of the preceding claims, **characterised in that** the solvent mixture contains ethylene glycol, diethyleneglycol, propylene glycol, glycerol, sorbitol, polyethyleneglycol and/or polypropyleneglycol.

8. Composition according to claim 7, **characterised in that** the solvent is a mixture of 20 to 60 wt.% of water, 5 to 15 wt.% of propylene glycol and 10 to 30 wt.% of diethyleneglycol monoethyl ether, based on the total weight of the composition in each case.

9. Composition according to one of the preceding claims, **characterised in that** it contains diclofenac or a salt thereof, preferably diclofenac sodium, in an amount of from 0.5 to 20 wt.%, based on the total weight of the composition.

10. Composition according to one of the preceding claims, **characterised in that** it contains other conventional pharmaceutical excipients and/or carriers and optionally other active substances.

11. Composition according to one of the preceding claims in the form of a solution for rubbing in, a skin spray, gel or impregnated plaster.

12. Process for preparing a composition according to one of the preceding claims, wherein diclofenac or one of the pharmaceutically acceptable salts thereof is dissolved in solvent and the phase thus obtained is mixed with water and the solubiliser, optionally dissolved in solvent.

## Revendications

1. Préparation à base de diclofénac ou d'un de ses sels pharmaceutiquement acceptables, pour le traitement topique d'inflammations et de douleurs contenant un solvant et au moins un agent solubilisant, qui contient comme agent solubilisant au moins un phospholipide, et dans laquelle le solvant est un mélange d'eau, de monoéthyléther de diéthylèneglycol et, éventuellement, de polyalcools en C₂-C₆ ainsi que de polyéthers de ces derniers, leurs esters et éthers, ainsi que de glycérides et/ou de dérivés éthoxylés de ces derniers.

2. Préparation selon la revendication 1, dans laquelle le phospholipide est un phospholipide naturel, en particulier de la lécithine d'oeuf ou de soja, un phospholipide modifié par voie enzymatique ou chimique, en particulier la lécithine hydrogénée, ou un phospholipide synthétique, en particulier la dipalmitoylphosphatidylcholine.

3. Préparation selon la revendication 1 ou 2, dans laquelle le phospholipide est exempt de matière grasse.

4. Préparation selon l'une quelconque des revendications précédentes, dans laquelle le phospholipide est contenu dans une proportion de 0,5 à 20% en poids par rapport au poids total de la préparation.

5. Préparation selon l'une quelconque des revendications précédentes, qui contient, comme autre agent solubilisant, au moins une des substances suivantes, esters et éthers, éventuellement éthoxylés, de polyols, en particulier esters d'acides gras de polyéthylèneglycolsorbitan, esters d'acides gras de sorbitan, esters d'acides gras de polyéthylèneglycol, éthers d'alcools gras de polyéthylèneglycol, et esters partiels d'acides gras de polyols.

6. Préparation selon l'une quelconque des revendications précédentes, qui contient l'agent solubilisant dans une proportion de 0,5 à 25 % en poids par rapport au poids total de la préparation.

7. Préparation selon l'une quelconque des revendications précédentes, dans laquelle le mélange de solvants contient de l'éthylèneglycol, du diéthylèneglycol, du propylèneglycol, de la glycérine, du sorbitol, du polyéthylèneglycol et/ou du polypropylèneglycol.

8. Préparation selon la revendication 7, dans laquelle le solvant est un mélange de 20 à 60 % en poids d'eau, 5 à 15 % en poids de propylèneglycol, et 10 à 30 % en poids de monoéthyléther de diéthylèneglycol, à chaque fois par rapport au poids total de la préparation.

9. Préparation selon l'une quelconque des revendications précédentes contenant du diclofénac ou son sel, de préférence du diclofénac-sodium, dans une proportion de 0,5 à 20 % en poids par rapport au poids total de la préparation.

10. Préparation selon l'une quelconque des revendications précédentes, qui contient d'autres adjuvants et/ou supports pharmaceutiques courants ainsi que, éventuellement, d'autres substances actives.

11. Préparation selon l'une quelconque des revendications précédentes sous forme d'une solution à appliquer par frottement, d'un spray à pulvériser sur la peau, d'un gel ou d'un emplâtre imbibé.

12. Procédé de fabrication d'une préparation selon l'une quelconque des revendications précédentes, dans lequel on dissout le diclofénac ou un de ses sels pharmaceutiquement acceptables dans un solvant et on mélange la phase ainsi obtenue avec de l'eau et, éventuellement, un agent solubilisant dissous dans le solvant.
